# EUROPÄISCHE PATENTSCHRIFT

(11) **EP 1 230 398 B1**
(45) Veröffentlichungstag und Bekanntmachung des Hinweises auf die Patenterteilung: **30.06.2004**
(21) Anmeldenummer: 99963245.8
(22) Anmeldetag: 16.11.1999
(51) Int. Cl.: C12Q 1/68, C07H 21/00

(54) **FARBSTOFFMARKIERTES OLIGONUKLEOTID ZUM MARKIEREN EINES NUKLEINSÄUREMOLEKÜLS**
COLOUR LABELLED OLIGONUCLEOTIDE FOR LABELLING A NUCLEIC ACID MOLECULE
OLIGONUCLEOTIDE MARQUE PAR COLORANT POUR LE MARQUAGE D'UNE MOLECULE D'ACIDE NUCLEIQUE

(43) Veröffentlichungstag der Anmeldung: 14.08.2002
(73) Patentinhaber: Atto-tec GmbH, 57076 Siegen (DE)
(72) Erfinder: SAUER, Markus, D-69124 Heidelberg (DE); WOLFRUM, Jürgen, D-37124 Rosdorf-Obernjesa (DE)
(74) Vertreter: Köllner & Brunotte
(86) Internationale Anmeldenummer: PCT/DE1999/003635
(87) Internationale Veröffentlichungsnummer: WO 2001/036668

(56) Entgegenhaltungen:
- EP-A- 0 745 690
- WO-A-98/10096
- SEIDEL C: "Nucleoside-specific quenching of fluorescent dyes. Nucleobase one-electron potentials and their correlation with static and dynamic quenching efficiencies" JOURNAL OF PHYSICAL CHEMISTRY, Bd. 100, Seiten 5541-53, XP000925372
- SAUER M ET AL: "Dynamics of the electron transfer reaction between an oxazine dye and DNA oligonucleotides monitored on the single-molecule level " CHEMICAL PHYSICS LETTERS, Bd. 284, Nr. (3,4), 1998, Seiten 153-63, XP000925373
- TYAGI S. ET AL: 'Multicolor molecular beacons for allele discrimination' NATURE BIOTECHNOLOGY Bd. 16, Nr. 1, 1998, Seiten 49 - 53

## Beschreibung

Die Erfindung bezieht sich auf ein farbstoffmarkiertes Oligonukleotid zum Markieren eines einen Zielsequenz-Abschnitt aufweisenden Nukleinsäuremoleküls, wobei das farbstoffmarkierte Oligonukleotid folgende Komponenten aufweist: einen Schleifenabschnitt, der eine zur Zielsequenz im wesentlichen komplementäre Schleifensequenz aufweist; einen an einem Ende des Schleifenabschnitts angeordneten ersten Stielabschnitt mit mindestens drei Nukleosiden; einen am anderen Ende des Schleifenabschnitts angeordneten zweiten Stielabschnitt mit mindestens drei Nukleosiden, wobei die beiden Stielabschnitte intramolekular hybridisieren können; und einen Fluorophor, der an einer Position des ersten Stielabschnitts gebunden ist.

Die Erfindung bezieht sich ferner auf zwei Verfahren zum Nachweisen eines einen Zielsequenz-Abschnitt aufweisenden Nukleinsäuremoleküls in einer Lösung.

Die eingangs genannten farbstoffmarkierten Oligonukleotide werden häufig "Nukleinsäuresonden" genannt. Sie spielen eine zentrale Rolle bei der schnellen und empfindlichen Detektion spezifischer, bekannter Nukleinsäuremoleküle (DNA oder RNA) in biologischen Proben in der Molekularbiologie und Biotechnologie. Zu speziellen Anwendungen gehören unter anderem die medizinische Früherkennung einer bakteriellen oder viralen Infektion, die Forensik, der Einsatz in der DNA/RNA-Amplifizierung durch PCR oder durch andere Techniken, in der Frühdiagnose eines genetischen Fehlers sowie bei der Diskriminierung zwischen ähnlichen Organismen und Allelen.

Es sind verschiedene Verfahren zur Erkennung und Mengenbestimmung von Nukleinsäuren bekannt. Das weit verbreitete Southern-Blotting-Verfahren zeichnet sich durch zeitraubende Arbeitsschritte und eine schlechte Empfindlichkeit aus.

Ein neueres, elegantes Verfahren zum Nachweisen eines spezifischen Nukleinsäuremoleküls verwendet die sogenannten "Molecular Beacons" (Tyagi et al. 1996, Nature Biotechnology 14, 303-308; Kostrikis et al. 1998, Science 279, 1228-1229). Molecular Beacons sind farbstoffmarkierte Oligonukleotide, die die eingangs genannte Stiel-Schleifen-Struktur haben. An den beiden freien Enden der Stielabschnitte (dem 3'- und dem 5'-Ende) ist jeweils ein Fluorophor gekoppelt. Der eine Fluorophor dient als Fluoreszenz-Farbstoff und der andere als Quencher-Farbstoff, der die Fluoreszenz des Fluoreszenz-Farbstoffs bei hinreichender räumlicher Nähe durch Foerster-Enerqietransfer löscht.

WO-A-98 10096 offenbart ein farbstoffmarkiertes Oligonukleotid, dessen Fluoreszenz-Farbstoff und Quencher-Farbstoff kein FRET-Paar bilden.

Die Sequenzen der Stielabschnitte an beiden Enden der Molecular Beacons sind derart gewählt, daß dann, wenn sich der Molecular Beacon faltet, die Stielabschnitte ausschließlich aneinander, nicht aber mit anderen Abschnitten des Oligonukleotids hybridisieren. Im Zustand der hybridisierten Stielabschnitte ist der Abstand zwischen dem Fluoreszenz-Farbstoff und dem Quencher-Farbstoff hinreichend klein, so daß der Fluoreszenz-Farbstoff auch bei geeigneter Anregung mit Licht nicht fluoresziert.

Der Schleifenabschnitt weist eine Sequenz auf, die zur Sequenz des Zielsequenz-Abschnitts komplementär ist. Befinden sich die Molecular Beacons und die Zielsequenz aufweisende DNA/RNA-Moleküle gemeinsam in einer Lösung, so können die Schleifenabschnitte und die Zielsequenz-Abschnitte hybridisieren. Die Sequenzen und Längen der Stiel- und Schleifenabschnitte sind derart gewählt, daß sich der Molecular Beacon unter Lösung der Hybridisierung der beiden Stielabschnitte entfaltet. Infolge der Entfaltung wird der räumliche Abstand zwischen dem Fluoreszenz-Farbstoff und dem Quencher-Farbstoff stark vergrößert. Der Fluoreszenz-Farbstoff kann dann zur Fluoreszenz angeregt werden.

Beobachtet man kontinuierlich die Fluoreszenz-Intensität des Fluoreszenz-Farbstoffs, so kann ein Anstieg festgestellt werden, wenn die Molecular Beacons die Zielsequenz-Abschnitte der Nukleinsäuremoleküle aufspüren und an diesen hybridisieren. Auf diese Weise können die Nukleinsäuremoleküle quantitativ nachgewiesen werden.

Der Nachteil dieser Art von Molecular Beacons liegt in ihrer relativ aufwendigen Synthese, da das Oligonukleotid sowohl am 5'- als auch am 3'-Ende spezifisch mit dem Fluoreszenz- bzw. dem Quencher-Farbstoff markiert werden muß.

Der Erfindung liegt die Aufgabe zugrunde, den Nachweis eines Nukleinsäuremoleküls mit Hilfe eines farbstoffmarkierten Oligonukleotids zu verbessern.

Diese Aufgabe wird erfindungsgemäß durch ein farbstoffmarkiertes Oligonukleotid mit den Merkmalen des Anspruchs 1, sowie durch ein Verfahren mit den Merkmalen des Anspruchs 10 bzw. 11 gelöst.

Die Erfindung geht von der Erkenntnis aus, daß die Fluoreszenz verschiedener Fluorophore durch Nukleoside über einen photoinduzierten Elektronentransfer gelöscht werden kann (Sauer et al. 1995, J. Fluoresc. 5, 247 - 261; Seidel et al. 1996, J. Phys. Chem. 100, 5541 - 5553). Die Effizienz der Fluoreszenzlöschung durch photoinduzierten Elektronentransfer hängt stark vom Abstand zwischen dem Fluorophor und dem Nukleosid ab, d.h. nur bei einem geringen Abstand zwischen Fluorophor und dem geeigneten Nukleosid (in einem Einzel- oder Doppelstrang) tritt eine spürbare Fluoreszenzlöschung auf. Es wurde festgestellt, das beispielsweise ein Guanosin, das mehr als 4 Basen von der Kopplungsstelle des Fluorophors entfernt ist, keinen merklichen Einfluß auf die Fluoreszenzfähigkeit des Fluorophors hat, sofern der Fluorophor nur über einen sehr kurzen Spacer an das zugehörige Nukleosid gebunden ist. Dies gilt sowohl für ein Guanosin im Strang des Fluorophors als auch für solche im gegenüberliegenden Strang.

Bei dem erfindungsgemäßen farbstoffmarkierten Oligonukleotid weist der zweite Stielabschnitt mindestens ein Quencher-Nukleosid auf, welches die Fluoreszenz des Fluorophors bei hinreichender räumlicher Nähe zwischen Fluorophor und Quencher-Nukleosid durch photoinduzierten Elektronentransfer löscht. Die Sequenz des ersten Stielabschnitts und die Position des Fluorophors sind derart gewählt, daß im hybridisierten Zustand der beiden Stielabschnitte eine für eine Fluoreszenzlöschung hinreichende räumliche Nähe zwischen dem Fluorophor und dem Quencher-Nukleosid vorliegt, und daß bei Hybridisierung des Schleifenabschnitts mit dem Zielsequenz-Abschnitt und Auflösung der Hybridisierung der Stielabschnitte keine Fluoreszenzlöschung des Fluorophors auftritt.

Ein solches farbstoffmarkiertes Oligonukleotid (bzw. eine solche Nukleinsäuresonde) hat eine Reihe von Vorteilen. Es wird nur ein einziger Fluorophor benötigt. Dadurch vereinfacht sich die Synthese. Da der Mechanismus der Fluoreszenzlöschung durch photoinduzierten Elektronentransfer genau verstanden ist, kann auch eine gezielte Optimierung der Fluoreszenzlöschung vorgenommen werden.

Für eine effiziente Löschung müssen Nukleosid und Fluorophor aufeinander abgestimmt sein. Von den natürlich vorkommenden Nukleosiden hat Guanosin die stärkste löschende Wirkung auf Rhodamin-Farbstoffe. Die Löscheffizienz kann dadurch erhöht werden, daß 7-Deaza-Guanosin als Quencher-Nukleosid verwendet wird. In beiden Fällen bietet es sich an, daß alle weiteren Nukleoside des zweiten Stielabschnitts Guanosine sind. Entsprechend können als Nukleoside des ersten Stielabschnitts Cytidine gewählt werden.

Die Sequenz des ersten Stielabschnitts kann derart gewählt werden, daß der erste Stielabschnitt nicht mit einem an den Zielsequenz-Abschnitt angrenzenden Abschnitt des Nukleinsäuremoleküls hybridisieren kann. Andernfalls könnte der Fluorophor des ersten Stielabschnitts in die Nähe eines als Quencher wirkenden Guanosins geraten.

Als weiteres Quencher-Nukleosid kann 7-Deaza-Adenosin verwendet werden. Durch Einsatz von 7-Deaza-Adenosin als Quencher wird der Löscheffekt auf den Fluorophor im Vergleich zum unmodifizierten Guanosin drastisch gesteigert. Wenn 7-Deaza-Adenosin als Quencher-Nukleosid eingesetzt wird, wird das Fluorophor vorteilhafterweise an Thymidin (im Falle von DNA-Molekülen) oder an Uridin (im Falle von RNA-Molekülen) gekoppelt. Diese Nukleoside gehen bei einer Hybridisierung der beiden Stielabschnitte eine Basenpaarung mit dem 7-Deaza-Adenosin ein.

Verwendet man 7-Deaza-Adenosin als Quencher-Nukleosid am zweiten Stielabschnitt und koppelt den Fluorophor an Thymidin oder Uridin, so liegt bei einer Hybridisierung des ersten Stielabschnitts mit einem Abschnitt des Nukleinsäuremoleküls dem Fluorophor im Doppelstrang ein natürliches, unmodifiziertes Adenosin gegenüber. Dieses läßt die Fluoreszenz des Fluorophors im wesentlichen unbeeinflußt. Daher kann eine Hybridisierung des ersten Stielabschnitts mit einem an den Zielsequenz-Abschnitt angrenzenden Abschnitt des Nukleinsäuremoleküls zugelassen werden, sofern die Sequenzen des ersten Stielabschnitts und des Zielsequenz-Abschnitts derart gewählt werden, daß sich kein Guanosin in der Nähe des Fluorophors befindet. Durch die Hybridisierung des ersten Stielabschnitts an einem Abschnitt des Nukleinsäuremoleküls befindet sich das Fluorophor in einer wohldefinierten Umgebung. Unkontrollierbare Hybridisierungen mit anderen Nukleinsäuremolekülen in der Lösung werden dadurch vermieden.

Guanosin, 7-Deaza-Guanosin und 7-Deaza-Adenosin können auch gemischt im zweiten Stielabschnitt verwendet werden.

Eine besonders einfache Synthese des farbstoffmarkierten Oligonukleotids kann dann erreicht werden, wenn der erste Stielabschnitt am 5'-Ende des Schleifenabschnitts angeordnet ist und der Fluorophor terminal am endständigen Nukleosid gekoppelt ist.

Eine weitere vorteilhafte Möglichkeit eröffnet sich, wenn der erste Stielabschnitt am 3'-Ende des Schleifenabschnitts angeordnet ist, der Fluorophor terminal am endständigen Nukleosid des ersten Stielabschnitts gekoppelt ist und das 5'-Ende des zweiten Stielabschnitts für eine Immobilisierung funktionalisiert ist. Auf diese Weise können die Nukleinsäuresonden z.B. auf einem DNA-Chip immobilisiert werden. Letzterer kann eine Hybridisierung durch ein Fluoreszenzsignal anzeigen.

Das 5'-Ende des zweiten Stielabschnitts kann mit einem Acrylamid-Molekül funktionalisiert werden. Eine solcherart funktionalisierte Nukleinsäuresonde kann bei der Herstellung eines Polyacrylamidgels durch Copolymerisation immobilisiert werden. Dies kann an einer bestimmten Position in einem Plattengel oder einer Kapillare erfolgen. Eine zu untersuchende Probe, die Nukleinsäuremoleküle mit den unterschiedlichsten Sequenzen enthalten kann, wird dann unter nicht-denaturierenden Bedingungen im Gel aufgetrennt. Das Vorhandensein eines den Zielsequenz-Abschnitt aufweisenden Nukleinsäuremoleküls zeigt sich durch ein entsprechendes Signal an der Position der immobilisierten Nukleinsäuresonde in Gel. Anschließend kann das den Zielsequenz-Abschnitt aufweisende Nukleinsäuremoleküls z.B. durch Ausschneiden aus dem Plattengel gezielt isoliert werden.

Der Schleifenabschnitt muß lang genug sein, um bei Hybridisierung mit dem Zielsequenz-Abschnitt die Hybridisierung der beiden Stielabschnitte aufzulösen. Er muß andererseits aber nur genau so lang sein, daß eine eindeutige Identifizierung des Zielsequenz-Abschnitts gegeben ist. Vorteilhafterweise umfaßt daher der Schleifenabschnitt 8 bis 50 Nukleoside.

Die beiden Stielabschnitte müssen mindestens so lang sein, daß eine zuverlässige Hybridisierung auftreten kann. Andererseits sollte die Hybridisierung der beiden Stielabschnitte jedoch im Falle der Hybridisierung des Schleifenabschnitts mit dem Zielsequenz-Abschnitt aufgelöst werden. Die Stärke der Hybridisierung läßt sich durch die Länge der beiden Stielabschnitte beeinflussen. Vorteilhafterweise umfaßt daher der erste Stielabschnitt 3 bis 8 Nukleoside und der zweite Stielabschnitt mindestens so viele Nukleoside wie der erste Stielabschnitt.

Als Fluorophore eignen sich prinzipiell alle bekannten Farbstoffmoleküle, speziell aber Rhodamin- und Phenoxazin-Farbstoffe. Letztere sind gut koppelbar und photostabil. Ein weiterer Vorteil des Einsatzes von Rhodamin- oder Phenoxazin-Farbstoffen besteht darin, daß als Anregungslichtquelle für eine Fluoreszenzdetektion kleine und billige Diodenlaser eingesetzt werden können.

Das erfindungsgemäße farbstoffmarkierte Oligonukleotid kann vorteilhaft zum Markieren eines einen Zielsequenz-Abschnitt aufweisenden Nukleinsäuremoleküls in einer Lösung verwendet werden, wobei das farbstoffmarkierte Oligonukleotid mit dem Nukleinsäuremolekül hybridisiert.

Das erfindungsgemäße farbstoffmarkierte Oligonukleotid ist außerdem besonders zum Nachweis eines einen Zielsequenz-Abschnitt aufweisenden Nukleinsäuremoleküls in einer Lösung geeignet. Dazu wird das Nukleinsäuremolekül mit einem erfindungsgemäßen farbstoffmarkierten Oligonukleotid markiert. Zur Stabilisierung des Doppelstrangs aus Sonde und Nukleinsäuremolekül und zur Verbesserung der Löscheffizienz zwischen Quencher und Fluorophor wird nach der Hybridisierung und vor der Aufnahme eines Nachweissignals der pH-Wert der Lösung auf Werte zwischen 2 und 4 eingestellt. Um zu vermeiden, daß sich Intensitätsschwankungen, beispielsweise aufgrund von Inhomogenitäten der Lösung, auf die Meßergebnisse auswirken, kann die Fluoreszenz des Fluorophors derart angeregt und detektiert werden, daß dessen Fluoreszenzabklingverhalten erfaßt wird.

Vorteilhafte Weiterbildungen der Erfindung sind in den Unteransprüchen gekennzeichnet.

Im folgenden wird die Erfindung anhand von Ausführungsbeispielen näher erläutert, die in den Figuren schematisch dargestellt sind. Gleiche Bezugsziffern in den einzelnen Figuren bezeichnen dabei gleiche Elemente. Im einzelnen zeigen:
- Fig. 1: ein farbstoffmarkiertes Oligonukleotid, bei dem die Stielabschnitte aneinander hybridisiert sind;
- Fig. 2: das farbstoffmarkierte Oligonukleotid gemäß Fig. 1, wobei der Schleifenabschnitt an einem Nukleinsäuremolekül hybridisiert ist;
- Fig. 3: ein zweites Ausführungsbeispiel eines farbstoffmarkierten Oligonukleotids, bei dem die Stielabschnitte aneinander hybridisiert sind;
- Fig. 4: das farbstoffmarkierte Oligonukleotid gemäß Fig. 3, wobei der Schleifenabschnitt an einem Nukleinsäuremolekül hybridisiert ist; und
- Fig. 5: ein drittes Ausführungsbeispiel eines farbstoffmarkierten Oligonukleotids, bei dem die Stielabschnitte aneinander hybridisiert sind;
- Fig. 6: das farbstoffmarkierte Oligonukleotid gemäß Fig. 5, wobei der Schleifenabschnitt an einem Nukleinsäuremolekül hybridisiert ist; und
- Fig. 7: eine schematische Darstellung der möglichen Zustandsänderungen beim photoinduzierten Elektronentransfer.

In den Figuren bezeichnen die Buchstaben A, C, G und T die Nukleoside Adenosin, Cytidin, Guanosin und Thymidin.

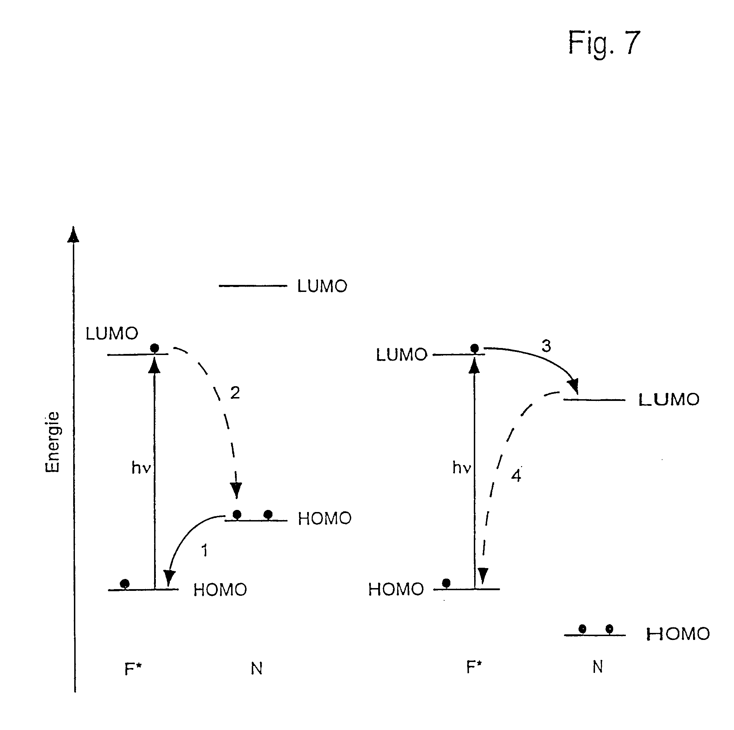

Im folgenden sei der photoinduzierte Elektronentransfer anhand von Fig. 7 kurz erläutert. Dargestellt ist die Fluoreszenzlöschung eines angeregten Farbstoff-Moleküls F* durch eine Nukleosid N. Die schwarzen Kreise repräsentieren Elektronen. Es sind jeweils das HOMO (highest occupied molecular orbital) und das LUMO (lowest unoccupied molecular orbital) eingezeichnet. Das HOMO ist das energetisch höchste, im elektronischen Grundzustand besetzte Molekülorbital. Das LUMO ist das energetisch niedrigste, im elektronischen Grundzustand unbesetzte Molekülorbital; es ist i.d.R. das Molekülorbital, das im ersten angeregten Zustand besetzt wird.

Prinzipiell gibt es zwei Möglichkeiten der Fluoreszenzlöschung durch photoinduzierten Elektronentransfer. Im in Fig. 7 links dargestellten Fall wirkt das Nukleosid N als Elektronenspender (Donor). Nach Anregung des Fluorophors F* geht ein Elektron vom doppelt besetzten HOMO des Nukleosids zum nun einfach besetzen HOMO des Fluorophors F* über (1). Es kommt zu einer Reduktion des angeregten Fluorophors F* durch das Nukleosid N. Das Elektron im LUMO des Fluorophors kann anschließend zum nun einfach besetzten HOMO des Nukleosid N übergehen (2). Dieser Fall tritt zwischen Guanosin und Rhodamin-Molekülen auf.

Im in Fig. 7 rechts dargestellten Fall wirkt das Nukleosid N als Elektronenakzeptor (Akzeptor). Aus dem einfach besetzten LUMO des angeregten Fluorophors F* geht das dort befindliche Elektron zum unbesetzten LUMO des Nukleosids N über (3). Es kommt zu einer Oxidation des angeregten Fluorophors F* durch das Nukleosid N. Das Elektron im LUMO des Nukleosids kann anschließend zum HOMO des Fluorophors zurückkehren (4).

In beiden Fällen kann das Elektron nach dem Elektronentransfer nicht mehr aus dem LUMO des angeregten Fluorophors F* durch Aussenden eines Photons in das HOMO zurückkehren. Der erste angeregte Zustand wurde strahlungslos deaktiviert. Die Fluoreszenz ist gelöscht.

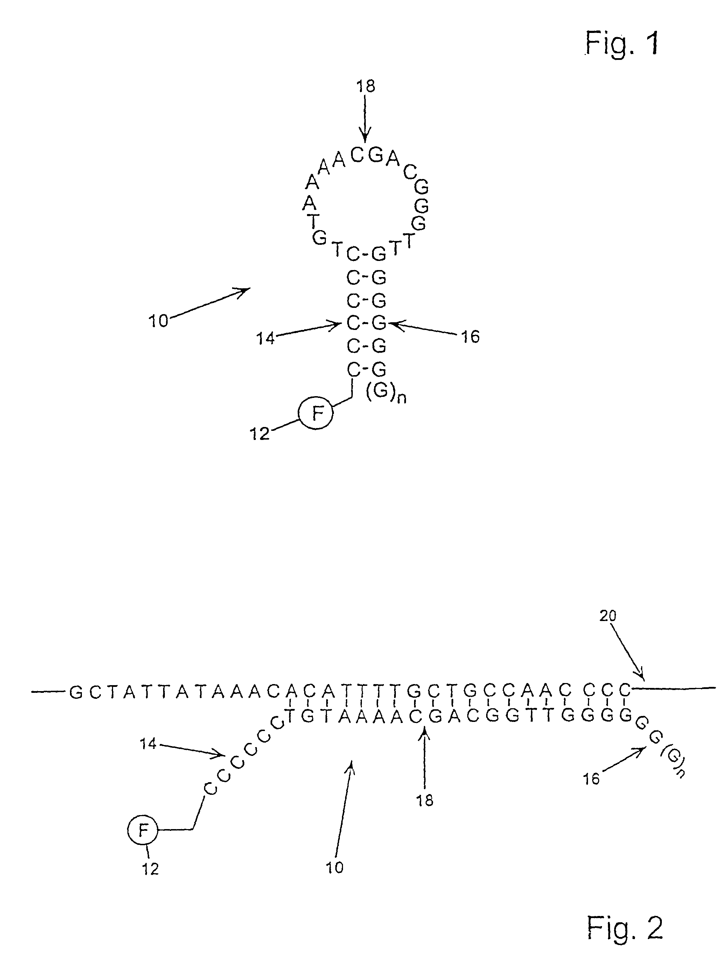

Fig. 1 zeigt ein Oligonukleotid 10, an dessen einem Ende ein Fluorophor 12 gekoppelt ist. Das Oligonukleotid 10 besteht aus einem ersten Stielabschnitt 14, einem zweiten Stielabschnitt 16 und einem Schleifenabschnitt 18. Die Sequenz des ersten Stielabschnitts 14 besteht aus 6 Nukleosiden, die alle Cytidine sind. Die Sequenz des zweiten Stielabschnitts 16 besteht aus mindestens 6 Guanosinen. Dadurch können der erste Stielabschnitt 14 und der zweite Stielabschnitt 16 aneinander hybridisieren und das Oligonukleotid 10 in eine Stiel-Schleifen-Struktur falten. Die genaue Länge des zweiten Stielabschnitts ist unerheblich, sofern er mindestens so viele Nukleoside aufweist, wie der erste Stielabschnitt.

Im folgenden wird auf Fig. 2 Bezug genommen. Die Sequenz des Schleifenabschnitts 18 ist derart gewählt, daß das Oligonukleotid 10 als Sonde für ein spezifisches Nukleinsäuremolekül 20 dienen kann. In der Regel ist die Schleifensequenz komplementär zur Sequenz eines Zielabschnitts des Nukleinsäuremoleküls 20. Werden das Oligonukleotid 10 und das Nukleinsäuremolekül 20 zusammen in eine Lösung gegeben, so hybridisiert der Schleifenabschnitt 18 am Zielsequenz-Abschnitt des Nukleinsäuremoleküls 20. Dadurch löst sich die Hybridisierung zwischen den beiden Stielabschnitten 14, 16. Infolgedessen vergrößert sich der Abstand zwischen dem Fluorophor 12 und den Guanosinen des zweiten Stielabschnitts 16. Letztere wirken nicht mehr fluoreszenzlöschend auf den Fluorophor 12, dessen Fluoreszenz somit beobachtet werden kann. Ein Anstieg der Fluoreszenz des Fluorophors 12 erlaubt daher qualitative und quantitative Aussagen über das Vorliegen des Nukleinsäuremoleküls 20.

Der Zielsequenz-Abschnitt auf dem Nukleinsäuremolekül 20 wird bei diesem Ausführungsbeispiel derart gewählt, daß der erste Stielabschnitt 14 bei Hybridisierung des Schleifenabschnitts 18 nicht mit dem Nukleinsäuremolekül 20 hybridisiert. Dadurch wird die Nähe zu irgendwelchen Guanosinen auf diesem Abschnitt des Nukleinsäuremoleküls 20 prinzipiell vermieden. In Fig. 2 erkennt man jedoch, daß der zweite Stielabschnitt 16 beispielsweise teilweise auf dem Nukleinsäuremolekül hybridisieren kann.

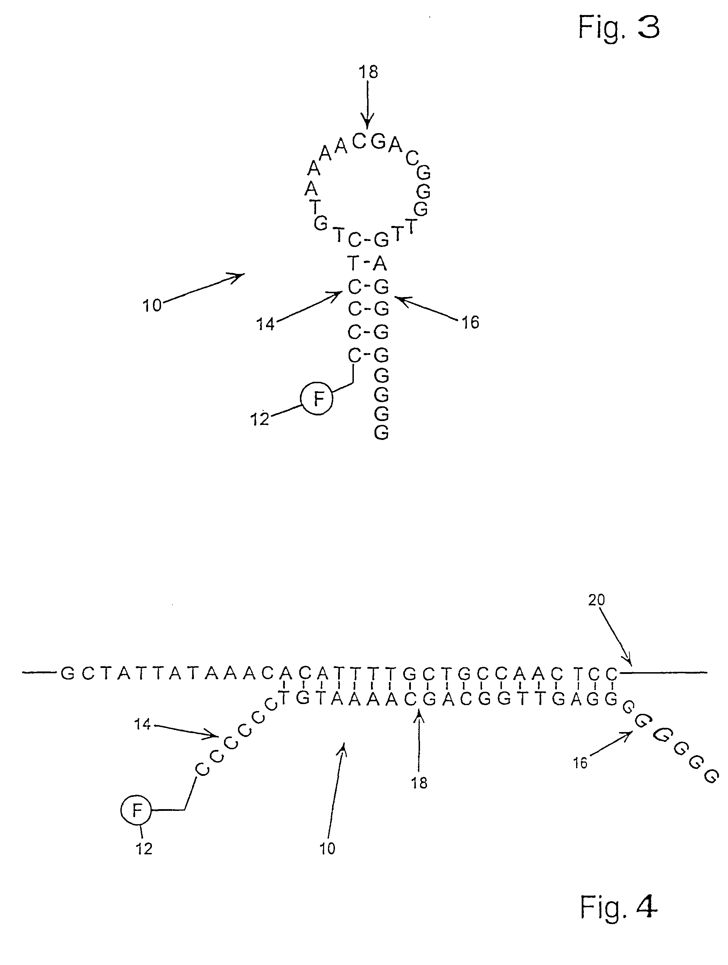

Im folgenden wird auf Fig. 3 Bezug genommen. Fig. 3 zeigt ein zweites Ausführungsbeispiel eines farbstoffmarkierten Oligonukleotids 10, das im wesentlichen mit dem Oligonukleotid gemäß Fig. 1 übereinstimmt. Das Oligonukleotid gemäß Fig. 3 weist jedoch im ersten Stielabschnitt 14 als fünftes Nukleosid - gezählt vom Ende her - ein Thymidin auf. Der zweite Stielabschnitt weist als neuntes Nukleosid - ebenfalls vom Ende her gezählt - ein Adenosin auf. Im hybridisierten Doppelstrang kommt es zwischen dem Adenosin und dem Thymidin zur Basenpaarung. Die Cytidine und Guanosine können dann nicht gegeneinander versetzt hybridisieren. Dadurch ist gewährleistet, daß an einem Ende ein Guanosin-Überstand resultiert, der die Löschung des Fluorophors begünstigt.

Fig. 4 zeigt das Oligonukleotid 10 gemäß Fig. 3 an einem Nukleinsäuremolekül 20 hybridisiert, das einen Zielsequenz-Abschnitt aufweist, dessen Sequenz komplementär zur Schleifensequenz ist.

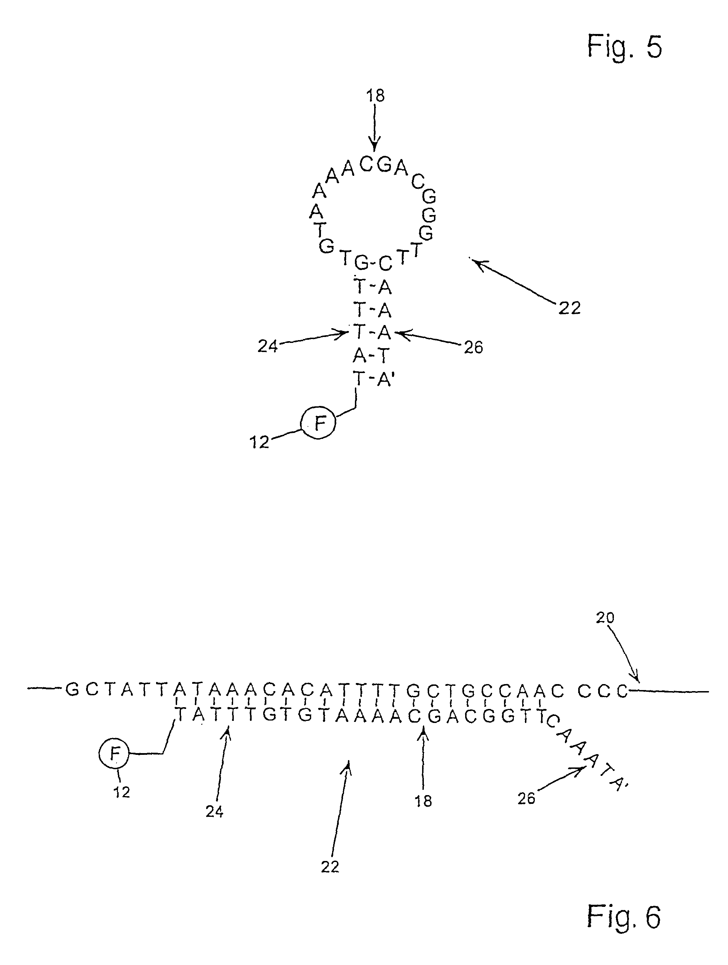

Im folgenden wird auf Fig. 5 Bezug genommen. Fig. 5 zeigt ein zweites Ausführungsbeispiel eines farbstoffmarkierten Oligonukleotids 22 mit einem ersten Stielabschnitt 24 und einem zweiten Stielabschnitt 26. Der zweite Stielabschnitt 26 weist genau 6 Nukleoside auf, von denen das endständige Nukleosid ein modifiziertes Adenosin ist, genauer gesagt 7-Deaza-Adenosin. Dies ist in den Fig. 5 und 6 mit A' bezeichnet. Entsprechend befindet sich an der im hybridisierten Zustand der beiden Stielabschnitte 24, 26 dem 7-Deaza-Adenosin gegenüberliegenden Stelle des ersten Stielabschnitts 24 ein Thymidin. An das Thymidin ist der Fluorophor 12 gekoppelt.

Fig. 6 zeigt das Nukleinsäuremolekül 20 gemäß Fig. 2, an welches das als Sonde dienende farbstoffmarkierte Oligonukleotid 22 gemäß Fig. 5 hybridisiert ist. Die Fig. 2 und 6 unterscheiden sich nur dahingehend, daß die Stielabschnitte 14, 16 bzw. 24, 26 unterschiedliche Sequenzen aufweisen. Der erste Stielabschnitt 24 hat in den Fig. 5 und 6 eine Sequenz, die eine Hybridisierung des ersten Stielabschnitts 24 mit einem an den Zielsequenz-Abschnitt angrenzenden Abschnitt des Nukleinsäuremoleküls 20 ermöglicht.

Der Zielsequenz-Abschnitt und die zugehörige Sequenz des Oligonukleotids 22 können in der folgenden Weise bestimmt werden:
a) Auf dem Nukleinsäuremolekül 20 wird ein Adenosin gesucht, bei dem sich unter den jeweils 4, links und rechts benachbarten Nukleosiden weder Cytidin noch Guanosin befindet.
b) In Verlängerung dieses Adenosins z.B. in 5'-Richtung des Nukleinsäuremoleküls 20 um mindestens 9 Nukleoside wird eine Sequenz gesucht, die das Nukleinsäuremolekül 20 eindeutig kennzeichnet.
c) Die Oligonukleotidsequenz 22 wird zu dieser Sequenz komplementär gebildet. Dabei bilden die ersten 3 bis 6 Nukleoside am 5'-Ende der Oligonukleotidsequenz die erste Stielsequenz 24.
d) Die zweite Stielsequenz 26 wird auf die folgende Weise gewonnen: Es wird geprüft, ob es am 3'-Ende der Oligonukleotidsequenz
   da) 3 bis 6 intramolekular ausschließlich zur ersten Stielsequenz komplementäre Nukleoside gibt, und ob
   db) das 3'-terminale Nukleosid Adenosin ist.

Ist dies der Fall, so bilden diese 6 Nukleoside die zweite Stielsequenz 26.

Ist dies nicht der Fall, so wird die Oligonukleotidsequenz um 3 bis 6 Nukleoside derart verlängert, daß sich 3 bis 6 intramolekular ausschließlich zur ersten Stielsequenz komplementäre Nukleoside mit 3'-terminalem Adenosin ergeben. (Sollte dies nicht möglich sein, da sich z.B. die Sequenz des ersten Stielabschnitts innerhalb der Zielsequenz wiederholt, muß ein anderes Adenosin gemäß Schritt a) gesucht werden.)

Das 3'-terminale Adenosin wird bei der Synthese des Oligonukleotids durch 7-Deaza-Adenosin ersetzt.

(Die minimale Zahl von 9 Nukleosiden in Schritt b) ergibt sich aus der minimalen Länge der Oligonukleotidsequenz bestehend aus 3 Nukleosiden für die beiden Stielsequenzen 24, 26 und mindestens 4 Nukleosiden für den Faltungsabschnitt des Oligonukleotids.)

Man sieht an diesem Beispiel, daß sich die Schleifenund Stielabschnitte auch überlappen können, und daß das farbstoffmarkierte Oligonukleotid 22 auch vollständig auf dem Nukleinsäuremolekül 20 hybridisieren kann.

Prinzipiell kann der Farbstoff sowohl an das 3'-Ende als auch an das 5'-Ende des Oligonukleotids gekoppelt werden. Hierzu stehen die folgenden Möglichkeiten zur Verfügung:
(a) bekannte Modifikation eines Endes des Oligonukleotids mit einer Aminfunktion, z.B. durch einen C6-Aminolinker, und anschließende Ankopplung des Farbstoffs an das modifizierte Ende über eine aktivierte Carboxylfunktion.
(b) synthetischer Einbau eines aminomodifizierten Nukleotids beim Aufbau des Oligonukleotids, z.B. in einem Synthesizer, und anschließende Ankopplung des Farbstoffs an das aminomodifizierte Nukleotid über eine aktivierte Carboxylfunktion.
(c) synthetischer Einbau des Farbstoffs als Phosphoramidit während der Oligonukleotid-Synthese.

Zur Optimierung der Löscheffizienz muß einerseits der durch die Hybridisierung gebildete Doppelstrang möglichst stabil sein. Dies wird in bekannter Weise durch Einstellen geeigneter Salzkonzentrationen erreicht. Andererseits kann aber auch der pH-Wert einen drastischen Einfluß auf die Löscheffizienz haben, etwa bei Verwendung eines Rhodamin-Farbstoffs, der eine freie Carboxylgruppe trägt, z.B. Tetramethylrhodamin. Durch die Protonierung der freien Carboxylfunktion im sauren Medium wird die Abstoßung zwischen dem Farbstoff und den Phosphatgruppen der Nukleotide verringert. Letzteres führt zu einem geringeren Abstand zwischen Farbstoff und Nukleotiden bzw. Nukleosiden und damit zu einer stärkeren Fluoreszenzlöschung. Bei Verwendung geeigneter Farbstoffe wird daher der pH-Wert vor Aufnahme eines Nachweissignals auf ca. 3 eingestellt.

Zum Nachweis des Nukleinsäuremoleküls wird die Fluoreszenz des Fluorophors vorzugsweise mit zeitkorreliertem Einzelphotonenzählen nachgewiesen (D.V. O'Connor und D. Phillips, "Time-correlated single photon counting", Adacemic Press, London, 1984). Neben der besonders hohen Empfindlichkeit bietet diese spektroskopische Technik den Vorteil, daß mit ihrer Hilfe das Fluoreszenzabklingverhalten des Fluorophors 12 beobachtet werden kann. Dieses hat sich als verläßlicheres Kriterium zum Nachweis der Fluoreszenz des Fluorophors 12 und damit des Nukleinsäuremoleküls 20 erwiesen als eine einfache Intensitätsmessung. Intensitätsschwankungen, beispielsweise aufgrund von Inhomogenitäten der Lösung, wirken sich dadurch nicht auf die Meßergebnisse aus.

Im Rahmen der Erfindung sind zahlreiche Abwandlungen und Weiterbildungen der beschriebenen Ausführungsbeispiele verwirklichbar. So muß beispielsweise der Fluorophor 12 nicht unmittelbar an demjenigen Nukleosid gekoppelt sein, das dem Quencher-Nukleosid im hybridisierten Zustand gegenüberliegt. Der Abstand zum erstgenannten Nukleosid muß nur hinreichend klein sein, um eine brauchbare Fluoreszenzlöschung durch das Quencher-Nukleosid hervorzurufen. Auch müssen der Schleifenabschnitt 18 und die Stielabschnitte 14, 16, 24, 26 nicht unmittelbar aneinander grenzen. Sie können durch weitere, kurze Sequenzabschnitte voneinander getrennt sein. Die Sequenz des Zielabschnitts und damit die komplementäre Sequenz des Schleifenabschnitts 18 sind prinzipiell beliebig. Außerdem kann das Nukleinsäuremolekül 20 ausschließlich aus dem Zielsequenz-Abschnitt bestehen.

## Patentansprüche

1. Farbstoffmarkiertes Oligonukleotid zum Markieren eines einen Zielsequenz-Abschnitt aufweisenden Nukleinsäuremoleküls, wobei das farbstoffmarkierte Oligonukleotid folgende Komponenten aufweist:
a) einen Schleifenabschnitt, der eine zur Zielsequenz im wesentlichen komplementare Schleifensequenz aufweist;
b) einen an einem Ende des Schleifenabschnitts angeordneten ersten Stielabschnitt mit mindestens drei Nukleosiden;
c) einen am anderen Ende des Schleifenabschnitts angeordneten zweiten Stielabschnitt mit mindestens drei Nukleosiden, wobei die beiden Stielabschnitte aneinander hybridisieren können; und
d) einen Fluorophor, der an einer Position des ersten Stielabschnitts gebunden ist;
**dadurch gekennzeichnet**,
e) daß der zweite Stielabschnitt mindestens ein Quencher-Nukleosid aufweist, welches Guanosin oder 7-Deaza-Guanosin oder 7-Deaza-Adenosin ist; und
f) wobei
fa) die Sequenz des ersten und zweiten Stielabschnitts,
fb) die Sequenz des Schleifenabschnitts ,
fc) die Position des Fluorophors derart gewählt sind und
fd) das Quencher-Nukleosid und der Fluorophor derart aufeinander abgestimmt sind,
fe) daß im hybridisierten Zustand der beiden Stielabschnitte eine für eine Fluoreszenzlöschung hinreichende räumliche Nähe zwischen dem Fluorophor und dem Quencher-Nukleosid vorliegt, und
ff) daß bei Hybridisierung des Schleifenabschnitte mit dem Zielsequenz-Abschnitt und Auflösung der Hybridisierung der Stielabschhitte keine Fluoreszenzlöschung des Fluorophors auftritt.

2. Farbstoffmarkiertes Oligonukleotid nach dem vorhergehenden Anspruch, **dadurch gekennzeichnet, daß** der erste Stielabschnitt derart gewählt ist, daß bei Hybridisierung des Schleifenabschnitts mit dem Zielsequenz-Abschnitt des Nukleinsäuremoleküls auch der erste Stielabschnitt mit einem Abschnitt des Nukleinsäuremoleküls hybridisiert.

3. Farbstoffmarkiertes Oligonukleotid nach einem der vorhergehenden Ansprüche, **dadurch gekennzeichnet,**
**daß** der erste Stielabschnitt am 5'-Ende des Schleifenabschnitts angeordnet ist; und
**daß** der Fluorophor terminal am endständigen Nukleosid des ersten Stielabschnitts gekoppelt ist.

4. Farbstoffmarkiertes Oligonukleotid nach Anspruch 1 oder 2, **dadurch gekennzeichnet,**
**daß** der erste Stielabschnitt am 3'-Ende des Schleifenabschnitts angeordnet ist;
**daß** der Fluorophor terminal am endständigen Nukleosid des ersten Stielabschnitts gekoppelt ist; und
**daß** das 5'-Ende des zweiten Stielabschnitts für eine Immobilisierung funktionalisiert ist.

5. Farbstoffmarkiertes Oligonukleotid nach Anspruch 4, **dadurch gekennzeichnet, daß** das 5'-Ende des zweiten Stielabschnitts mit einem Acrylamid-Molekül funktionalisiert ist.

6. Farbstoffmarkiertes Oligonukleotid nach einem der vorhergehenden Ansprüche, **dadurch gekennzeichnet, daß** der Schleifenabschnitt 8 bis 50 Nukleoside umfaßt.

7. Farbstoffmarkiertes Oligonukleotid nach einem der vorhergehenden Ansprüche, **dadurch gekennzeichnet, daß** der erste Stielabschnitt maximal 8 Nukleoside umfaßt.

8. Farbstoffmarkiertes Oligonukleotid nach einem der vorhergehenden Ansprüche, **dadurch gekennzeichnet, daß** der Fluorophor ein Rhodamin- oder Phenoxazin-Farbstoffmolekül aufweist.

9. Verfahren zum Nachweisen eines einen Zielsequenz-Abschnitt aufweisenden Nukleinsäuremoleküls in einer Lösung,
wobei ein farbstoffmarkiertes oligonukleotid nach einem der vorhergehenden Ansprüche mit dem Nukleinsäuremolekül hybridisiert; und
wobei nach der Hybridisierung und vor der Aufnahme eines Nachweissignals der pH-Wert der Lösung auf Werte zwischen 2 und 4 eingestellt wird.

10. Verfahren zum Nachweisen eines einen Zielsequenz-Abschnitt aufweisenden Nukleinsäuremoleküls in einer Lösung,
wobei ein farbstoffmarkiertes Oligonukleotid nach einem der Ansprüche 1 bis 8 mit dem Nukleinsäuremolekül hybridisiert; und
wobei danach die Fluoreszenz des Fluorophors derart angeregt und detektiert wird, daß dessen Fluoreszenzabklingverhalten erfaßt wird.

## Claims

1. A dye-labeled oligonucleotide for labeling a nucleic acid molecule having a target sequence section, which dye-labeled oligonucleotide has the following components:
a) a loop section which has a loop sequence essentially complementary to the target sequence;
b) a first stem section having at least three nucleosides and arranged at one end of the loop section;
c) a second stem section having at least three nucleosides and arranged at the other end of the loop section, with the two stem sections being able to hybridize with one another; and
d) a fluorophore which is bound to a position of the first stem section;
wherein
e) the second stem section has at least one quencher nucleoside which is guanosine or 7-deazaguanosine or 7-deazaadenosine; and
f) wherein
fa) the sequence of the first and second stem section,
fb) the sequence of the loop section,
fc) the position of the fluorophore
being chosen and
fd) the quencher nucleoside and the fluorophore are suited to one another
fe) such that in the hybridized state of the two stem sections the fluorophore and the quencher nucleoside are spatially close enough for a fluorescence quenching, and
ff) no fluorescence quenching of the fluorophore takes place during hybridization of the loop section with the target sequence section and breaking-up of the hybridization of the stem sections.

2. The dye-labeled oligonucleotide as claimed in the preceding claim, wherein the first stem section is chosen such that when the loop section hybridizes with the target sequence 'section of the nucleic acid molecule the first stem section, too, hybridizes with a section of said nucleic acid molecule.

3. The dye-labeled oligonucleotide as claimed in any of the preceding claims, wherein
the first stem section is arranged at the 5' end of the loop section; and
the fluorophore is coupled terminally to the terminal nucleoside of said first stem section.

4. The dye-labeled oligonucleotide as claimed in claims 1 or 2, wherein
the first stem section is arranged at the 3' end of the loop section;
the fluorophore is coupled terminally to the terminal nucleoside of said first stem section; and
the 5' end of the second stem section is functionalized for immobilization.

5. The dye-labeled oligonucleotide as claimed in claim 4, wherein the 5' end of the second stem section is functionalized with an acrylamide molecule.

6. The dye-labeled oligonucleotide as claimed in any of the preceding claims, wherein the loop section comprises 8 to 50 nucleosides.

7. The dye-labeled oligonucleotide as claimed in any of the preceding claims, wherein the first stem section comprises not more than 8 nucleosides.

8. The dye-labeled oligonucleotide as claimed in any of the preceding claims, wherein the fluorophore has a rhodamine or phenoxazine dye molecule.

9. A method for detecting in a solution a nucleic acid molecule having a target sequence section, wherein
a dye-labeled oligonucleotide according to one of the preceding claims hybridizes with the nucleic acid molecule; and,
after hybridization and prior to recording a detection signal, the pH of said solution is adjusted to values of between 2 and 4.

10. A method for detecting in a solution a nucleic acid molecule having a target sequence section, wherein
a dye-labeled oligonucleotide according to any of claims 1 to 8 hybridizes with the nucleic acid molecule; and,
thereafter, the fluorescence of the fluorophore is excited and detected such that the (fluorescence decay behavior thereof is recorded.

## Revendications

1. Oligonucléotide marqué par colorant, pour le marquage d'une molécule d'acide nucléique qui présente un tronçon de séquence voulue, l'oligonucléotide marqué par colorant présentant les composants suivants:
a) une portion en boucle qui présente une séquence de boucle essentiellement complémentaire de la séquence voulue,
b) une première portion en manche disposée à une extrémité de la portion en boucle et qui comprend au moins trois nucléosides,
c) une deuxième portion en manche disposée à l'autre extrémité de la portion en boucle, qui comprend au moins trois nucléosides, les deux portions en manche pouvant être hybridées l'une à l'autre et
d) un fluorophore qui est lié à une position de la première portion en manche,
**caractérisé en ce que**
e) la deuxième portion en manche présente au moins un nucléoside d'arrêt qui est la guanosine, la 7-déazaguanosine ou la 7-déazaadénosine et
f) dans lequel
fa) la séquence de la première et de la deuxième portion en manche,
fb) la séquence de la portion en boucle et
fc) la position du fluorophore sont sélectionnées de telle sorte et
fd) le nucléoside d'arrêt et le fluorophore sont accordés l'un à l'autre de telle sorte que
fe) lorsque les deux portions en manche sont à l'état hybridé, il existe entre le fluorophore et le nucléoside d'arrêt une proximité spatiale suffisante pour éteindre la fluorescence et que
ff) lors de l'hybridation de la portion en boucle avec la portion de séquence voulue et libération de l'hybridation des portions en manche, la fluorescence du fluorophore n'est pas éteinte.

2. Oligonucléotide marqué par colorant selon la revendication précédente, **caractérisé en ce que** la première portion en manche est sélectionnée de telle sorte que, lorsque la portion en boucle est hybridée avec la portion de séquence voulue de la molécule d'acide nucléique, la première portion en manche est également hybridée avec une portion de la molécule d'acide nucléique.

3. Oligonucléotide marqué par colorant selon l'une quelconque des revendications précédentes, **caractérisé en ce que** la première portion en manche est disposée à l'extrémité 5' de la portion en boucle et **en ce que** le fluorophore est couplé en position terminale au nucléoside terminal de la première portion en manche.

4. Oligonucléotide marqué par colorant selon la revendication 1 ou 2, **caractérisé en ce que** la première portion en manche est disposée à l'extrémité 3' de la portion en boucle, **en ce que** le fluorophore est couplé en position terminale au nucléoside terminal de la première portion en manche et **en ce que** l'extrémité 5' de la deuxième portion en manche est fonctionnalisée en vue d'une immobilisation.

5. Oligonucléotide marqué par colorant selon la revendication 4, **caractérisé en ce que** l'extrémité 5' de la deuxième portion en manche est fonctionnalisée avec une molécule d'acrylamide.

6. Oligonucléotide marqué par colorant selon l'une quelconque des revendications précédentes, **caractérisé en ce que** la portion en boucle comprend de 8 à 50 nucléosides.

7. Oligonucléotide marqué par colorant selon l'une quelconque des revendications précédentes, **caractérisé en ce que** la première portion en manche comprend au plus 8 nucléosides.

8. Oligonucléotide marqué par colorant selon l'une quelconque des revendications précédentes, **caractérisé en ce que** le fluorophore présente une molécule de colorant à la rhodamine ou à la phénoxazine.

9. Procédé de détection d'une molécule d'acide nucléique qui présente une portion de séquence voulue dans une solution, dans lequel un oligonucléotide marqué par colorant selon l'une quelconque des revendications précédentes est hybridé avec la molécule d'acide nucléique et dans lequel, après l'hybridation et avant la réception d'un signal de détection, le pH de la solution est réglé à des valeurs comprises entre 2 et 4.

10. Procédé de détection d'une molécule d'acide nucléique qui présente une portion de séquence voulue dans une solution, dans lequel un oligonucléotide marqué par colorant selon l'une quelconque des revendications 1 à 8 est hybridé avec la molécule d'acide nucléique et dans lequel la fluorescence du fluorophore est ensuite excitée et détectée de manière à enregistrer l'évolution de l'extinction de sa fluorescence.
